# EUROPEAN PATENT SPECIFICATION

(11) **EP 2 081 521 B1**
(45) Date of publication and mention of the grant of the patent: **18.06.2014**
(21) Application number: 07838442.7
(22) Date of filing: 18.09.2007
(51) Int. Cl.: A61F 2/856, A61F 2/915, A61F 2/954

(54) **SELF-EXPANDING SIDE BRANCH BIFURCATED STENT**
SELBSTEXPANDIERENDER GEGABELTER STENT MIT SEITENAST
STENT BIFURQUÉ À BRANCHE LATÉRALE AUTO - EXPANSIBLE

(30) Priority: 13.11.2006 US 858521 P; 12.09.2007 US 853947
(43) Date of publication of application: 29.07.2009
(73) Proprietor: Boston Scientific Limited, Christ Church (BB)
(72) Inventor: OZHERED, Irina, Plymouth, MN 55446 (US); MARTIN, Daryl, Maple Grove, MN 55369 (US); NODDIN, Richard, Elk River, MN 55330 (US)
(74) Representative: Hauck Patent- und Rechtsanwälte
(86) International application number: PCT/US2007/020230
(87) International publication number: WO 2008/060351

(56) References cited:
- WO-A-99/29262
- US-A1- 2002 193 873
- US-A1- 2003 045 923
- US-A1- 2006 100 686
- US-B1- 6 261 316

## Description

### BACKGROUND OF THE INVENTION

### Field of the Invention

This invention relates to a bifurcated stent.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. They may be self-expanding, expanded by an internal radial force, such as when mounted on a balloon, or a combination of self-expanding and balloon expandable (hybrid expandable). Stents may be implanted to prevent restenosis following angioplasty in the vascular system.

A complication arises when stenoses form at vessel bifurcation sites. A bifurcation site is an area of the vasculature or other portion of the body where a first (or parent) vessel is bifurcated into two or more branch vessels. Where a stenotic lesion or lesions form at such a bifurcation, the lesion(s) can affect only one of the vessels (i.e., either of the branch vessels or the parent vessel) two of the vessels, or all three vessels. Many prior art stents however are not wholly satisfactory for use where the site of desired application of the stent is juxtaposed or extends across a bifurcation in an artery or vein such, for example, as the bifurcation in the mammalian aortic artery into the common iliac arteries.

One aspect of this complication involves the non-uniform force vectors required to expand a bifurcated stent. While the main stent body of a bifurcated stent expands in a generally uniform and radial manner to assume a substantially tubular structure, the bifurcation branch requires at least one additional expansion vector which pushes it away from the main stent body. The requirement for and interaction between these multiple expansion vectors complicates the mechanics of stent expansion. Prior attempts to facilitate multiple expansion vectors included the use of multiple expansion balloons and balloons with expansion protuberances to push out the bifurcation branch.

The art referred to and/or described above is not intended to constitute an admission that any patent, publication or other information referred to herein is "prior art" with respect to this invention.

From document US 2002/0193873 A1 systems for delivering a bifurcated stent to a bifurcation site comprising catheters and/or bifurcated stents delivered therefrom have become known. The known stents comprise a generally tubular main stent body and a side-arm comprising a side branch assembly which in the unexpanded state lies along a circumferential layer of the main stent body.

From document US patent No. 6,261,316 B1 a deployment system and graft for deploying a bifurcated graft within both iliac branches have become known. The deployment system includes a delivery catheter having a releasable restraint for the main body section of the graft and separate releasable restraints for each of the first and second branch sections of the graft.

From document US 2003/0045923 A1 a stent comprising a tubular member having openings therein and having a plurality of interconnected members and one or more frangible restraining members have become known. The frangible restraining members break upon partial expansion of the stent. Thereafter, the stent self-expands.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

This invention contemplates a number of embodiments where any one, any combination of some, or all of the embodiments can be incorporated into a stent as claimed. The present invention is directed to a tether released self expanding bifurcated intravascular stent. The stent can assume a bifurcated configuration without the need for a dual or irregularly shaped expansion balloon. The tether can be intertwined in the petal or projecting members of the bifurcation to prevent premature expansion but can easily be removed to assure proper deployment. The stent can also have a mounting ring which loops around a side opening in the stent main body. The stent can also be at least partially, balloon expandable.

At least one embodiment of the inventive concept is directed to a tether which spans a portion of the side-arm projecting portion of the stent along a linear, curved, longitudinally collinear, longitudinally oblique, parallel contoured, or undulating path, or a combination of one or more path types. The tether can be at least partially positioned between the stent body and an inflation balloon and can be above, below, and/or intertwined through stent cells. The tether can extend circumferentially and/or longitudinally from the side branch assembly.

At least one embodiment of the inventive concept is directed to the tether having a weaker portion which is less resistant to tensional force than the rest of the tether. This weaker portion can be positioned facing the side opening. The weaker portion can be narrower than the other tether portions, can be pre-cut or perforated, and/or can be held together by a releasable adhesive. These and other aspects of the invention are set forth below.

### BRIEF DESCRIPTION OF THE SEVERAL VIEWS OF THE DRAWINGS

The invention is best understood from the following detailed description when read in connection with accompanying drawings, in which:
Figure 1 is a flat plan view of an unexpanded bifurcated stent with a restraining tether extending through the side branch assembly.
Figure 2 is a schematic perspective view of an expanded bifurcated stent.
Figure 3 is a cross sectional schematic view of a non-central region of the expanded bifurcated stent.
Figure 4 is a cross sectional schematic view of the central region of the expanded bifurcated stent.
Figure 5 is a flat plan view of an unexpanded bifurcated stent with a restraining tether extending through the side branch assembly in an alternative configuration.
Figure 6 is a lateral view of a cross section of a partially inflated balloon within a stent with a restraining tether interlaced above and beneath stent struts.
Figure 7 is a lateral view of a cross section of a partially inflated balloon within a stent with a restraining tether positioned below stent struts but above a side branch assembly.
Figure 8 is an overhead flat pan view of stent retainer.
Figure 9 is a perspective view of a stent retainer.

### DETAILED DESCRIPTION OF THE INVENTION

The invention will next be illustrated with reference to the figures wherein the same numbers indicate similar elements in all figures. Such figures are intended to be illustrative rather than limiting and are included herewith to facilitate the explanation of the apparatus of the present invention.

For the purposes of this disclosure, like reference numerals in the figures shall refer to like features unless otherwise indicated.

Depicted in the figures are various aspects of the invention. Elements depicted in one figure may be combined with, or substituted for, elements depicted in another figure as desired.

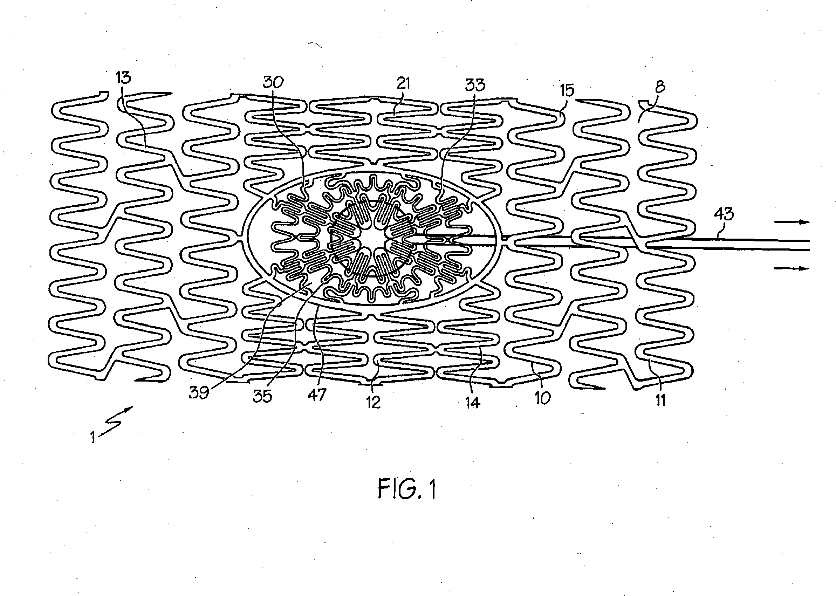

Referring now to Figure 1, there is shown an unexpanded bifurcated stent (1). The stent comprises two portions, a generally tubular main stent body (11) which defines a primary fluid lumen or a first lumen and a bifurcating side branch secondary body or side arm. The secondary body comprises a side branch assembly (30). The main stent body (11) comprises a distal region (13) comprising all of the main stent body distal to the side branch assembly (30), a proximal region (15) comprising all of the main stent body proximal to the side branch assembly (30), and a central region (21) comprising the region of the main stent body (11) between the distal and proximal regions. The main stent body (11) is constructed out of one or more suitable materials including but limited to steel, stainless steel, titanium, and nitinol. In at least one embodiment, the central region (21) comprises a welded material such as nitinol or spring steel which is engaged to stainless steel proximal and/or distal regions.

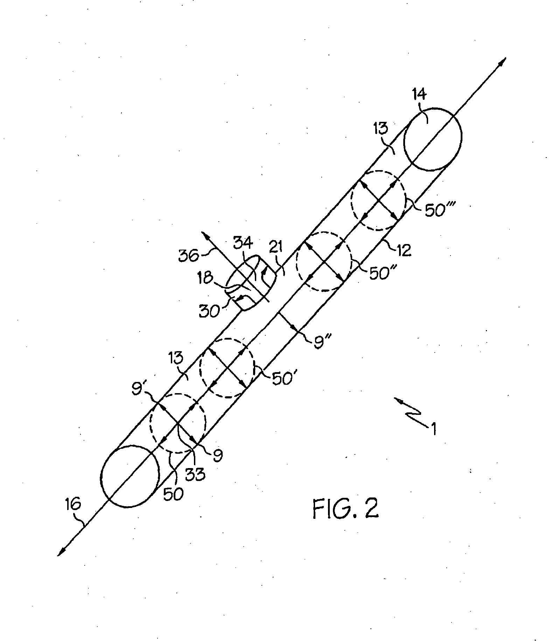

The stent (1) has an expanded and an unexpanded state. The main stent body (11) can be formed using suitable known techniques to assume its expanded state through self expansion, balloon inflation, or by any other method currently known in the art. When in the expanded state, the stent (1) assumes a greater volume than when in the unexpanded state. As shown in FIGs. 2, 3, and 4, when expanding, cross sections (50) of the stent located in either the distal or proximal regions expands from the application of radial force which may be represented as force vectors (9, 9', 9", 9"') directed at perpendicular angles relative to the center of the cross section (31). This allows for adequate expansion with the use of a uniformly expanding balloon and is facilitated by the fact that any counter resistance to expansion provided by a stent member (9) results in additional reflected expansive force applied to another stent member (9') located at the opposite side of the cross section.

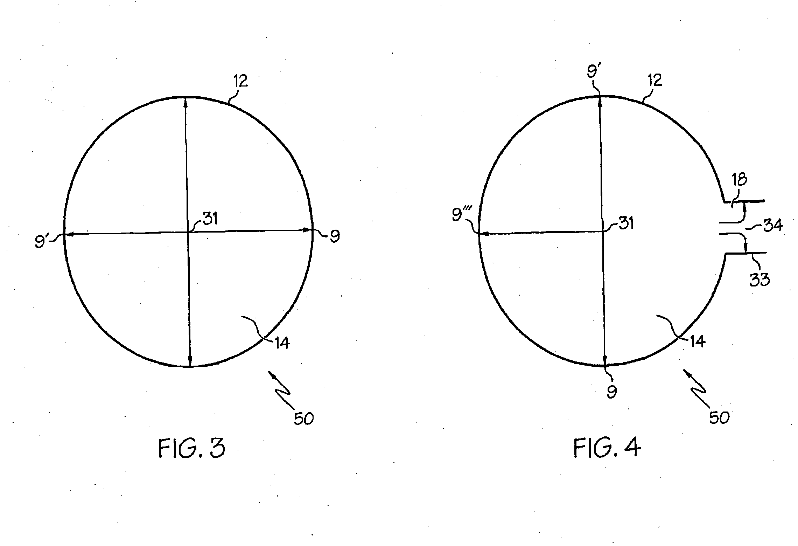

In contrast as shown in FIGs. 2 and 4, expansion in the central region (21) is more complex. In the central region (21), because the area of the side opening (18) does not have any solid material to push back against an expanding balloon, the distribution of the force vectors will not be uniform throughout the central region (21) and will differ from that at the portion of the cross section opposite it (9"'). In addition, the side branch assembly (30) expands along an arced path which is not symmetrical to the radially perpendicular angles that the other portions of the stent expand along. These differences complicate the deployment of a side branch assembly (30) and require a mechanism capable of coordinating these different expansion force vectors.

The inner surface of the main stent body (11) faces and defines a first fluid lumen (14). As shown in FIGs. 2, 3, and 4, the stent (1) comprises a plurality of cross sections, each cross section having a circumference. FIG. 2 illustrates that these circumferences together form a first circumferential layer (12) which is defined by the stent members (9) of the first stent body (10) and is the circumference of the main branch. In the unexpanded state, the side branch assembly (30) generally lies along the first circumferential layer (12) and covers at least a portion of a side opening (18) present in the main stent body (11). In the expanded state, at least a portion of the side branch assembly (30) bends, twists, and/or projects away from the first circumferential layer (12) and defines a secondary fluid lumen (34) in fluid communication with the primary fluid lumen (14).

The main stent body (11) comprises a plurality of stent members (9). In at least one embodiment, such as in the embodiment depicted in FIG. 1, the stent members (9) are interconnected expansion columns (7) each comprising one or more undulating struts (5). The struts (5) defme cells (8) which are the open spaces between the struts (5) and are positioned along the first circumferential layer (12). When the stent (1) is expanded from a radially compressed state to a radially expanded state, the relative configuration of the adjacent stent members (9) is altered.

The side branch assembly (30) is surrounded by stent members (9) and is engaged to at least one stent member (9). In at least one embodiment, the side branch assembly further comprises a mounting ring (47) which defines the perimeter of the side opening (18). The mounting ring (47) and/or the side opening (18) can be of any shape including but not limited to polygonal, circular, square, rectangular, elliptical, oval, or any combination thereof.

As shown in FIG. 2, when the side branch assembly (30) is deployed, it forms a generally tubular structure which extends along a second longitudinal axis (36) directed at an oblique angle relative to a first longitudinal axis (16) along which the main stent body (11) extends For the purposes of this application, the term "oblique" refers to an angle of greater than zero degrees, such as an angle of between about 1 and about 180 degrees. An oblique angle explicitly includes angles of about 90 degrees.

Referring again to FIG. 1, there is shown at least one embodiment in which the side branch assembly (30) comprises projecting members (33) which are biased to self expand, projecting away from the first circumferential layer (12). Before deploynient, these biased projecting members (33) are restrained from projecting away from the first circumferential layer (12) by one or more tethers (43) in contact with the side branch assembly (30). The tethers (43) are anchored in such a manner that they provide a pulling tension which counteracts the bias of the self expanding members (33).

When the side branch assembly (30) is properly positioned and ready for deployment, the tether (43) is retracted, releasing the biased projecting members (33). The tethers may be retracted by one or more suitable mechanisms including but not limited to the following: *1)* the tethers extend along at least a portion of the catheter and are pulled from at least a portion of the stent when appropriate and *2)* the tethers have a loop disposed about a hook on a stent member (9) or are otherwise engaged to the first stent body (11) and are pulled away from the side opening (18) as the first stent body (11) assumes the expanded state.

In order to properly project the side branch assembly (30), the tether (34) need only clear the expanding portion of the side branch assembly (30) and need not be completely removed from the stent (1). When the tethers are retracted from the projecting members (33), the projecting members (33) bend twist or otherwise project away from the first circumferential layer (12) and define the second fluid lumen (34).

The inventive concept contemplates embodiments in which the tethers (43) are constructed out of one or more suitable materials strong enough to structurally withstand both the pulling tension present when the stent (1) is in the unexpanded state as well as the retraction force applied to release the tethers (43) from the side branch assembly (30). Such suitable materials include but are not limited to: metals, polymers, composites, multiple fibers arranged or braided together to form a cable like configurations, rigid materials, flexible materials, wires, surgical suture materials or the like, biodegradable materials, and any combination thereof. In some cases and in particular in the case of a tether comprising one or more biodegradable materials, it is contemplated by this inventive concept that the retraction force may sever one or more portions of the tether (43) and one or more portions of the severed tether (43) will remain engaged to the stent (1) in the expanded state. In at least one embodiment, one or more of the severed tether portions remaining engaged to the stent (1) in the expanded state, remains so engaged at least until the remaining tether portion is at least partially biodegraded.

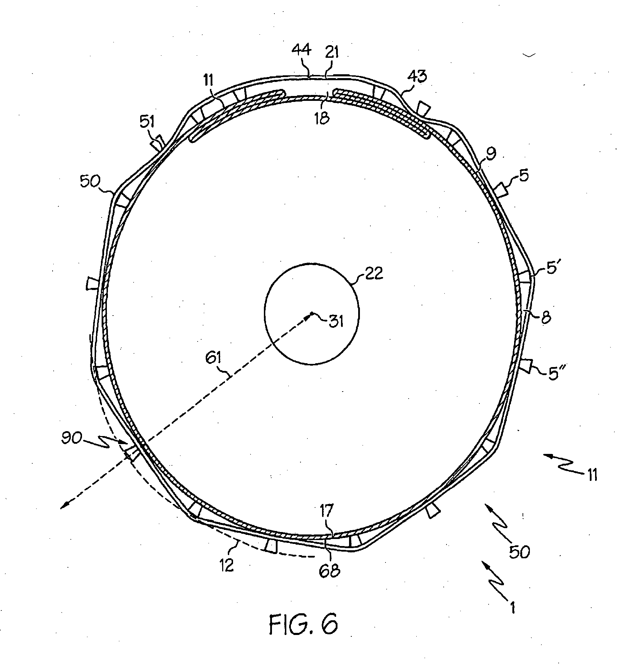

In at least one embodiment, the projecting members (33) may be one or more petals arranged in an iris or flattened arrangement when in an unexpanded state. When projecting away from the first circumferential layer (12) to assume the expanded state, the iris opens in a "petal-like" manner to form a rounded or crown like arrangement which defines the second fluid lumen (34). When in the iris arrangement, the petals have a plurality of petal struts (35) which define open spaces or petal cells (39) in between the petal struts (35). The one or more tethers (43) wend through the petal cells (39) with at least a portion of the tether (43) extending across the outer surface of the petals and at least a portion of the tether (43) extending across the inner surface of the petals. FIG. 6 illustrates an embodiment where the tether (43) is alternately positioned above or beneath the circumferential layer (12) by its location relative to immediately adjacent stent struts (5').

Embodiments of the inventive concept disclosed in FIGs. 1-5 also include tethers (43) extending through or across other locations on the stent (1) including but not limited to the following: The tether (43) is engaged to stent members (9) of the main stent body; the tether wends through the cells (8) of the main stent body (11) extending along both the outer and inner surfaces of the main stent body (11); the stent members (9) or mounting ring (47) comprise a loop smaller than a cell (8) to which the tether (43) is tied, fastened, welded, or otherwise engaged. Similarly, the tether (43) can span in any number of directions including but not limited to from a more proximal position to a more distal position or vice versa, along an axis not parallel to the first longitudinal axis (16), and along a nonlinear path. In at least one embodiment, at least a portion of the tether (43) wends along the side branch assembly along a curved path. The curved path could generally mirror the contour of the mounting ring (47) or side opening.

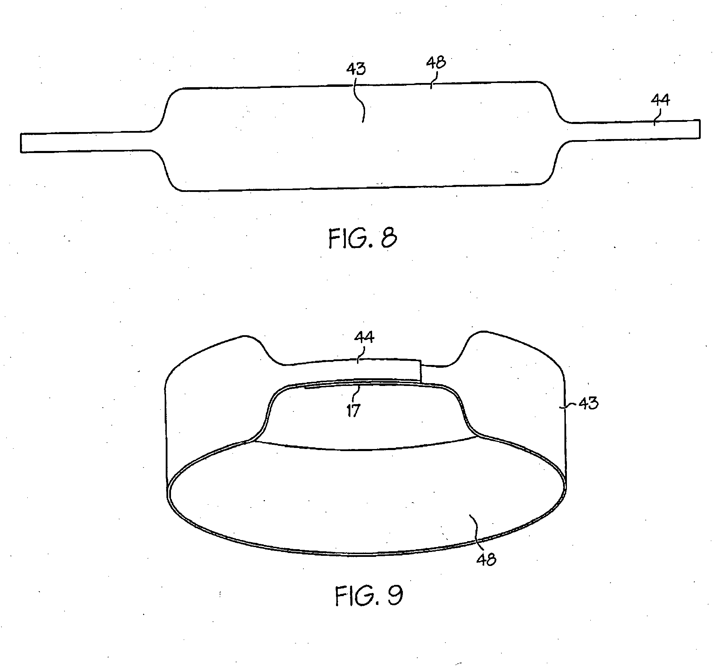

For purposes of this application the definition of the term "tether" is a length of rope, filament, thread, wire, or other tensile article having a narrow or wide width relative to the length which restrains the motion of one object relative to another object. This definition of "tether" explicitly includes retaining lengths of material having variable widths such as those illustrated in FIGs. 8 and 9.

Embodiments of the inventive concept illustrated in FIGs. 1-5 further include tethers (43) which extend completely across the side branch assembly (30) as well as tethers (43) which only partially cross the side branch assembly (30). In at least one embodiment, the tether (43) extends from a first position along the side opening (18) or mounting ring (47) along a generally linear path progressing closer to the center of the side opening, at some point however the tether curves to generally mirror the contour of the mounting ring (47) or side opening and then again assumes a generally linear path extending to a position on the opposite side of the side opening or mounting ring (47) as the first position, for instance as shown in FIG. 1.

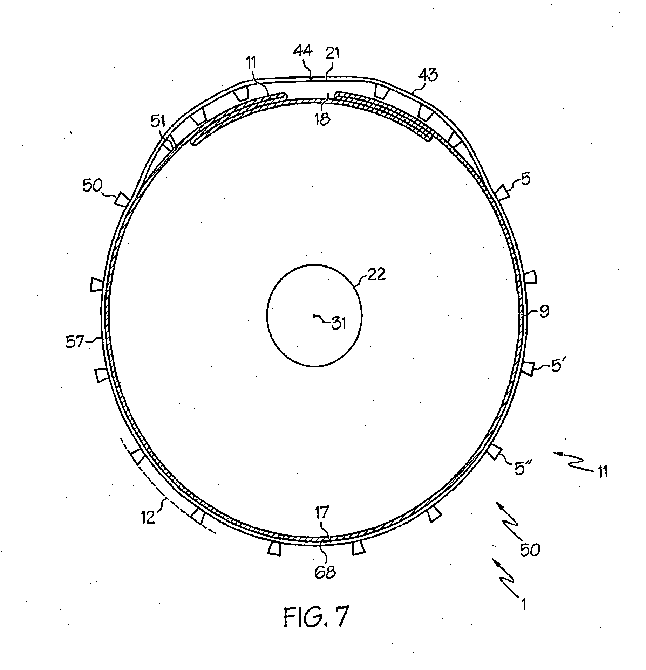

Referring now to FIGs 6 and 7 there are shown cross sections (50) of a tether restrained bifurcated stent (1) in which the tether (43) is at least partially wrapped about the circumferential layer (12) of a stent (1). In at least one embodiment (as shown in FIG. 6), the tether (43) is pulled so taut as to defme at least one substantially linear spans which whose center extends at an approximately perpendicular angle (90) to a radial axis (61) extending radially from the center point (31) of the stent (1) or catheter (22) to the circumferential layer (12). In another embodiment (as shown in FIG. 7), at least a portion of the tether is less taut and defines one or more curved arcs (57). The curved arcs (57) can generally mirror the path of the circumferential layer (12) or can deviate from that path.

FIG. 6 illustrates a cross section (50) in which at least a portion of a tether (43) is weaved through cells (8) by traversing above the outer surface (5o) and beneath the inner surface (5i) of immediately adjacent stent struts (5' and 5"). In at least one embodiment, the stent is at least partially disposed about an inflation balloon (9) and the tether can at least partially be wedged in place between the inner surface of the struts (5i) and an inflation balloon (9). FIG. 7 illustrates a cross section (50) in which at least a portion of the tether (43) is positioned beneath stent struts (5). The tether (43) can be positioned at any position along the length of the stent (1) including at the longitudinal position of the side branch opening (18). In at least one embodiment, the tethers are at least partially positioned over the side branch assembly.

Referring now to both FIGs. 6 and 7, there is shown at least one embodiment where the tether (43) comprises a weakened portion (44). This weakened portion (44) is less resistant to particularly directed pressing forces than other portions of the tether length (43). When the stent (1) is expanded to a particular width or when the side branch assembly exerts a pre-determined level of pressing force against the tether (43), the integrity of the weakened portion (44) fails and at least partially frees the side branch assembly to transition from the iris to the crown configuration. The weakened position (44) can be positioned directly opposite the side branch opening (18) or immediately opposite a folded portion (11) of a non-fully inflated balloon (9).

Illustrated in FIGs. 8 and 9 is at least one embodiment wherein the weakened portion (44) of the tether (43) is narrower than the non-weakened portion (48) and is located at least at some other portion of the tether (43) length. This narrower area is weaker because it has less material to resist the expansive pressing force. In addition, the wider area of non-weakened portion (48) can distribute the pressing counter-forces more widely across the surface area of an expansion balloon or stent member. This wider distribution of counter-force also reduces overall stress against the balloon or stent.

Also illustrated in FIGs. 8 and 9 is at least one other embodiment where the weakened portion (44) comprises two separate pieces of material which are bonded together. FIG. 8 shows the two portions before they are bonded and FIG. 9 shows them after they are bonded. This bonding can be accomplished by an adhesive layer (17) between the two portions, by heat methods, or by any other techniques known in the art which provides bonded materials a structural integrity which is weaker than the structural integrity of integrated wholes of the same material. The weakened portion (44) can be designed such that a pre-determined level of expansive pressure will separate the bonded pieces of material at the weakened portion (44) while being insufficient to separate other portions of the tether.

Other embodiments contemplated by this inventive concept are one or more tethers (43) with at least one pre-cut, perforated (21 in FIGs. 6 and 7), or otherwise scarred portions of the tether length (43), which is a weaker portion (44) than the non-weakened portions (48) of the tether (43). In addition as shown in FIG. 6 and 7, the non-weakened portion (48) can be further reinforced by its engagement to the balloon (9) or stent member (5) by a layer of adhesive material (17) or any other form of reinforcing engagement known in the art. In at least one embodiment the tether (43) is bonded by an adhesive at the circumferential opposite (68) (opposite side of a diameter line) from the side branch opening (18). A tether with a wider portion (48) that is also bonded by an adhesive (17) to the stent (1) or to the balloon (9) will also be further reinforced by the greater surface area available for the adhesive to bond along. In at least one embodiment the bonded tether is at least partially composed of a biodegradable polymer fiber. In embodiments where the tether is bonded to an inflation balloon, after stent expansion when the balloon is deflated and withdrawn from the expanded stent, the adhesive bond pulls the tethers away from the stent with the balloon.

One method of assembling a tethered bifurcated stent involves the steps of collapsing an un-inflated balloon, folding the tether over the collapsed balloon, extending the narrower portions of the tethers radially from the collapsed balloon, positioning the stent over the collapsed balloon with the narrow portions extending through one or two cells, bonding together the narrow portions either over or under some or all of the stent struts or side branch assembly.

In at least one embodiment, a tether (43) extends from a first position along the side opening or mounting ring (47) and back to either the same or an opposite point but does not completely cross the side branch assembly (30). As shown in FIG. 1, the tether (43) can curve along the side branch assembly (30) and exit at substantially the same side or position along the side opening (18) or mounting ring (47) as it entered from.

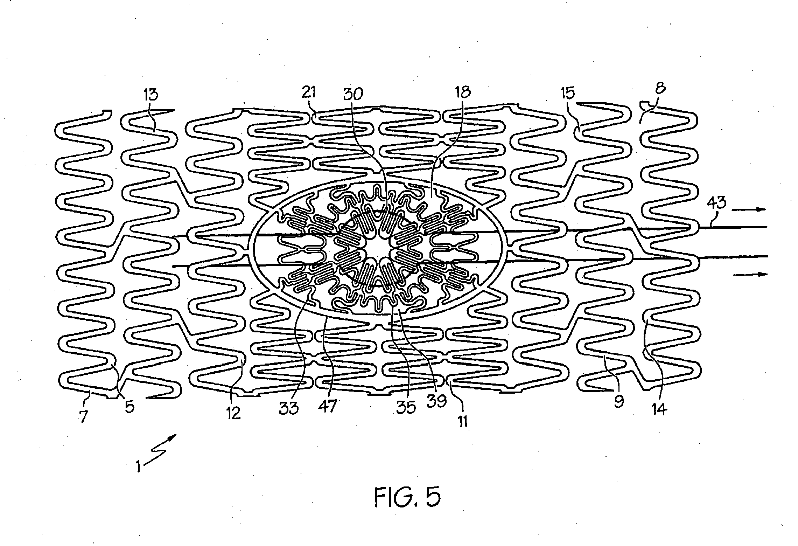

In at least one embodiment as shown in FIG. 5, a pair of tethers (43) are emplaced along the side branch assembly (30) and cross the perimeter of the side opening (18) or the mounting ring (47) at opposite ends of the side branch assembly (30). This design can assure that the various projecting members (33) become disengaged from the tether (43) relatively simultaneously. As a result, improper deployment that could occur if one portion of the side branch assembly (30) were to self expand while another portion were to remain restrained by the tether (43) is avoided.

By restraining the side branch assembly (30) with a tether (43), the projection of the second stent body can be accomplished independent of the expansion of the main stent body (11). An example of the utility of independent projection can be seen in circumstances where the medical protocol requires the side branch assembly (30) to be projected prior to stent expansion. In such a circumstance the tethers (43) can be pulled from the side branch assembly (30) before the stent main body (11) undergoes expansion. Similarly, if the medical protocol indicates that the main body be secured in the main vessel lumen before the side branch assembly (30) is projected into the side lumen, the tethers (43) can remain in place until appropriate.

In some embodiments the stent, its delivery system, or other portion of an assembly may include one or more areas, bands, coatings, members, etc. that are detectable by imaging modalities such as X-Ray, MRI, ultrasound, etc. In some embodiments at least a portion of the stent and/or adjacent assembly is at least partially radiopaque.

In some examples at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

The therapeutic agent can be at least one or various types of therapeutic agents including but not limited to: at least one restenosis inhibiting agent that comprises drug, polymer and bio-engineered materials or any combination thereof. In addition, the coating can be a therapeutic agent such as at least one drug, or at least one other pharmaceutical product such as non-genetic agents, genetic agents, cellular material, etc. Some examples of suitable non-genetic therapeutic agents include but are not limited to: at least one anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate. It will be appreciated that other types of coating substances, well known to those skilled in the art, can be applied to the stent (1) as well.

This completes the description of the preferred and alternate embodiments of the invention. The above disclosure is intended to be illustrative and not exhaustive. This description will suggest many variations and alternatives to one of ordinary skill in this art. The various elements shown in the individual figures and described above may be combined, substituted, or modified for combination as desired. All these alternatives and variations are intended to be included within the scope of the claims where the term "comprising" means "including, but not limited to".

## Claims

1. A bifurcated stent having an unexpanded state in which the stent comprises a generally tubular main stent body (11) defining a first lumen comprising a plurality of cross sections (50) forming a first circumferential layer (12), and an expanded state in which said first lumen is radially enlarged and a side-arm projects obliquely outward from the first lumen, the side-arm defining a second lumen in fluid communication with the first lumen, said side-arm comprising a side branch assembly (30) comprising projecting members (33) biased to self-expand and to project away from the first circumferential layer (12), wherein in the unexpanded state, the side branch assembly (30) lies along the first circumferential layer (12) and covers at least a portion of a side opening (18) present in the main stent body (11),
**characterized in that**
in at least the unexpanded state the side-arm projecting members (33) of the stent are restrained from self-expansion by at least one tether (43) which is releasable during stent deployment so as to allow formation of the side-arm independent of the expansion of the main stent body (11).

2. The stent of claim 1 in which the side branch assembly (30) of the stent comprises a plurality of projecting struts which project away from the circumferential layer by bending, a plurality of projecting cells are defined by open spaces located between the plurality of projecting struts.

3. The stent of claim 1 in which the tether (43) spans a portion of the side branch assembly of the stent along a path configured according to one geometry selected from the list consisting of: a linear path, a curved path, a path collinear to the longitudinal axis of the stent, a path oblique to the longitudinal axis of the stent, a path generally corresponding to the contours of an outer circumference of the side branch assembly (30) of the stent, a curved path, an undulating path, and any combination thereof.

4. The stent of claim 1 which in the unexpanded state further comprises an inflation balloon, at least a portion of the tether (43) is positioned between the inflation balloon and the generally tubular wall, that portion of the tether (43) positioned on the opposite side of a cross sectional diameter of the stent from the side branch assembly (30) of the stent is engaged to the inflation balloon by an adhesive.

5. The stent of claim 2 in which the plurality of projecting struts have an inner surface which in the unexpanded state faces the first lumen and an oppositely facing outer surface, at least a portion of the tether (43) extending across a portion of the outer surface and at least a portion of the tether (43) extending across a portion of the inner surface.

6. The stent of claim 1 in which at least one tether (43) is positioned beneath the generally tubular wall and above the side branch assembly (30) of the stent.

7. The stent of claim 1 in which the tether (43) comprises two ends, in the unexpanded state neither end of the tether (43) being positioned within the side branch assembly (30) of the stent.

8. The stent of claim 1 wherein in the unexpanded state at least a portion of the side branch assembly (30) of the stent is biased to project obliquely outward from the first lumen, the pushing force of the bias being less than a restraining opposite force vector applied by the tether (43).

9. The stent of claim 1 in which the stent further comprises a mounting ring (47) having a circumference and defining a side opening in the generally tubular wall, the side branch assembly of the stent being engaged to the mounting ring (47) being positioned within the circumference of the mounting ring (47) and in which the mounting ring (47) further comprises an inner surface facing the inner lumen and an oppositely facing outer surface wherein the tether (43) extends across at least a portion of the outer surface of the mounting ring (47).

10. The stent of claim 1 in which the tether (43) extends at least partially in a circumferential direction about the stent.

11. The stent of claim 10 in which the tether (43) forms a complete loop about the stent.

12. The stent of claim 1 in which the tether (43) does not extend completely across the side branch assembly (30) of the stent.

13. The stent of claim 1 in which at least a portion of the generally tubular wall comprises interconnected stent strut members having an inner stent surface which faces the first lumen and an oppositely facing outer stent surface wherein stent cells define open spaces between the interconnected stent strut members and the tether (43) extends through at least one stent cell.

14. The stent of claim 1 in which the tether (43) extends from a more distal position on the side branch assembly (30) of the stent to a more distal position on the side branch assembly (30) of the stent.

15. The stent of claim 1 in which a portion of the tether (43) is a weaker portion, being less resistant to tensional force than the other portions of the tether (43).

## Patentansprüche

1. Gegabelter Stent, der einen nicht expandierten Zustand aufweist, in dem der Stent einen im Wesentlichen röhrenförmigen Haupt-Stentkörper (11) umfasst, der ein erstes Lumen bestimmt, das eine Vielzahl Querschnitte (50) umfasst, die eine erste Umfangsschicht (12) bilden, und einen expandierten Zustand, in dem das erste Lumen radial vergrößert ist und ein Seitenarm schräg nach außen von dem ersten Lumen hervorsteht, wobei der Seitenarm ein zweites Lumen in Fluidverbindung mit dem ersten Lumen bestimmt, wobei der Seitenarm eine Seitenastbaugruppe (30) umfasst, die hervorstehende Elemente (33) umfasst, die vorgespannt sind, von selbst zu expandieren und weg von der ersten Umfangsschicht (12) hervorzustehen, wobei in dem nicht expandierten Zustand die Seitenastbaugruppe (30) entlang der ersten Umfangsschicht (12) liegt und mindestens einen Abschnitt einer in dem Haupt-Stentkörper (11) vorhandenen Seitenöffnung (18) verdeckt,
**dadurch gekennzeichnet, dass**
zumindest in dem nicht expandierten Zustand die hervorstehenden Seitenarmelemente (33) des Stents durch mindestens eine beim Einsetzen des Stents lösbare Befestigung (43) vom Selbstexpandieren zurückgehalten werden, um unabhängig vom Expandieren des Haupt-Stentkörpers (11) das Bilden eines Seitenarms zu erlauben.

2. Stent nach Anspruch 1, bei dem die Seitenastbaugruppe (30) des Stents eine Vielzahl hervorstehender Streben umfasst, die durch Biegen von der Umfangsschicht ausgehend hervorstehen, eine Vielzahl hervorstehender Zellen ist durch offene Räume bestimmt, die sich zwischen der Vielzahl hervorstehender Streben befinden.

3. Stent nach Anspruch 1, bei dem die Befestigung (43) einen Abschnitt der Seitenastbaugruppe des Stents entlang eines Pfads umspannt, der gemäß einer Geometrie ausgestaltet ist, die aus der Liste bestehend aus einem linearen Pfad, einem gekrümmten Pfad, einem zu der Längsachse des Stents kollinearen Pfad, einem zu der Längsachse des Stents schräg verlaufenden Pfad, einem im Wesentlichen den Konturen eines äußeren Umfangs der Seitenastbaugruppe (30) des Stents entsprechenden Pfad, einem gekrümmten Pfad, einem wellenförmigen Pfad oder einer beliebigen Kombination daraus, ausgewählt ist.

4. Stent nach Anspruch 1, der im nicht expandierten Zustand überdies einen Inflationsballon umfasst, mindestens ein Abschnitt der Befestigung (43) ist zwischen dem Inflationsballon und der im Wesentlichen röhrenförmigen Wandung angeordnet, wobei der Abschnitt der Befestigung (43), der auf der gegenüberliegenden Seite eines Querschnitts-Durchmessers des Stents von der Seitenastbaugruppe (30) des Stents angeordnet ist, durch einen Kleber mit dem Inflationsballon in Eingriff gebracht ist.

5. Stent nach Anspruch 2, bei dem die Vielzahl hervorstehender Streben eine innere Oberfläche, die im nicht expandierten Zustand dem ersten Lumen zugewandt ist, und eine entgegengesetzt gewandte äußere Oberfläche aufweist, wobei mindestens ein Abschnitt der Befestigung (43) sich über einen Abschnitt der äußeren Oberfläche erstreckt und mindestens ein Abschnitt der Befestigung (43) sich über einen Abschnitt der inneren Oberfläche erstreckt.

6. Stent nach Anspruch 1, bei dem mindestens eine Befestigung (43) unter der im Wesentlichen röhrenförmigen Wandung und über der Seitenastbaugruppe (30) des Stents angeordnet ist.

7. Stent nach Anspruch 1, bei dem die Befestigung (43) zwei Enden umfasst, wobei im nicht expandierten Zustand keines der Enden der Befestigung (43) innerhalb der Seitenastbaugruppe (30) des Stents angeordnet ist.

8. Stent nach Anspruch 1, wobei im nicht expandierten Zustand mindestens ein Abschnitt der Seitenastbaugruppe (30) des Stents vorgespannt ist, schräg nach außen von dem ersten Lumen hervorzustehen, wobei die schiebende Kraft der Vorspannung geringer ist als ein entgegengesetzter zurückhaltender Kraftvektor, der durch die Befestigung (43) ausgeübt wird.

9. Stent nach Anspruch 1, bei dem der Stent überdies einen Montagering (47) umfasst, der einen Umfang aufweist und eine Seitenöffnung in der im Wesentlichen röhrenförmigen Wandung bestimmt, wobei die Seitenastbaugruppe des Stents mit dem Montagering (47) in Eingriff ist, wenn sie innerhalb des Umfangs des Montagerings (47) angeordnet ist, und bei dem der Montagering (47) überdies eine dem inneren Lumen zugewandte innere Oberfläche und eine entgegengesetzt gewandte äußere Oberfläche umfasst, wobei die Befestigung (43) sich über mindestens einen Abschnitt der äußeren Oberfläche des Montagerings (47) erstreckt.

10. Stent nach Anspruch 1, bei dem die Befestigung (43) sich zumindest teilweise in einer Umfangsrichtung um den Stent herum erstreckt.

11. Stent nach Anspruch 10, bei dem die Befestigung (43) eine vollständige Schleife um den Stent herum bildet.

12. Stent nach Anspruch 1, bei dem die Befestigung (43) sich nicht vollständig über die Seitenastbaugruppe (30) des Stents erstreckt.

13. Stent nach Anspruch 1, bei dem mindestens ein Abschnitt der im Wesentlichen röhrenförmigen Wandung miteinander verbundene Stentstrebenelemente umfasst, die eine innere Stentoberfläche, die dem ersten Lumen zugewandt ist, und eine entgegengesetzt gewandte äußere Stentoberfläche aufweisen, wobei Stentzellen offene Räume zwischen den miteinander verbundenen Stentstrebenelementen bestimmen und die Befestigung (43) sich durch mindestens eine Stentzelle erstreckt.

14. Stent nach Anspruch 1, bei dem die Befestigung (43) sich von einer distaleren Position an der Seitenastbaugruppe (30) des Stents zu einer distaleren Position an der Seitenastbaugruppe (30) des Stents erstreckt.

15. Stent nach Anspruch 1, bei dem ein Abschnitt der Befestigung (43) ein schwächerer Abschnitt ist, der weniger widerstandsfähig gegenüber Spannkraft ist als die anderen Abschnitte der Befestigung (43).

## Revendications

1. Stent bifurqué ayant un état non expansé dans lequel le stent comprend un corps de stent principal (11) généralement tubulaire définissant une première lumière comprenant une pluralité de sections transversales (50) formant une première couche circonférentielle (12), et un état expansé dans lequel ladite première lumière est agrandie radialement et un bras latéral fait saillie obliquement vers l'extérieur à partir de la première lumière, le bras latéral définissant une deuxième lumière en communication fluidique avec la première lumière, ledit bras latéral comprenant un ensemble de branche latérale (30) comprenant des éléments en saillie (33) qui sont pressés pour s'auto-expanser et pour faire saillie à partir de la première couche circonférentielle (12), dans lequel, dans l'état non expansé, l'ensemble de branche latérale (30) est étendu le long de la première couche circonférentielle (12) et couvre au moins une portion d'une ouverture latérale (18) présente dans le corps de stent principal (11),
**caractérisé en ce que**,
au moins dans l'état non expansé, les éléments en saillie (33) du bras latéral du stent sont empêchés de s'auto-expanser par au moins une attache (43) qui peut être relâchée pendant le déploiement du stent de façon à permettre la formation du bras latéral indépendamment de l'expansion du corps de stent principal (11).

2. Stent selon la revendication 1, dans lequel l'ensemble de branche latérale (30) du stent comprend une pluralité d'entretoises en saillie qui font saillie à partir de la couche circonférentielle par flexion, une pluralité de cellules en saillie sont définies par des espaces ouverts situés entre la pluralité d'entretoises en saillie.

3. Stent selon la revendication 1, dans lequel l'attache (43) enjambe une portion de l'ensemble de branche latérale du stent le long d'un passage configuré conformément à une géométrie sélectionnée dans la liste constituée de : un passage linéaire, un passage incurvé, un passage colinéaire à l'axe longitudinal du stent, un passage oblique par rapport à l'axe longitudinal du stent, un passage correspondant généralement aux contours d'une circonférence extérieure de l'ensemble de branche latérale (30) du stent, un passage incurvé, un passage ondulé, et toute combinaison de ceux-ci.

4. Stent selon la revendication 1 qui, dans l'état non expansé, comprend également un ballonnet de gonflage, au moins une portion de l'attache (43) est positionnée entre le ballonnet de gonflage et la paroi généralement tubulaire, la portion de l'attache (43) positionnée sur le côté opposé d'un diamètre de section transversale du stent à partir de l'ensemble de branche latérale (30) du stent est mise en prise avec le ballonnet de gonflage par un adhésif.

5. Stent selon la revendication 2, dans lequel la pluralité d'entretoises en saillie ont une surface intérieure qui, dans l'état non expansé, fait face à la première lumière, et une surface extérieure dirigée dans le sens opposé, au moins une portion de l'attache (43) s'étendant à travers une portion de la surface extérieure, et au moins une portion de l'attache (43) s'étendant à travers une portion de la surface intérieure.

6. Stent selon la revendication 1, dans lequel au moins une attache (43) est positionnée au-dessous de la paroi généralement tubulaire et au-dessus de l'ensemble de branche latérale (30) du stent.

7. Stent selon la revendication 1, dans lequel l'attache (43) comprend deux extrémités, aucune extrémité de l'attache (43) n'étant, dans l'état non expansé, positionnée à l'intérieur de l'ensemble de branche latérale (30) du stent.

8. Stent selon la revendication 1, dans lequel, dans l'état non expansé, au moins une portion de l'ensemble de branche latérale (30) du stent est pressée pour faire saillie obliquement vers l'extérieur à partir de la première lumière, la force de poussée de la pression étant inférieure à un vecteur de force opposé de retenue appliqué par l'attache (43).

9. Stent selon la revendication 1, dans lequel le stent comprend également une bague de montage (47) ayant une circonférence et définissant une ouverture latérale dans la paroi généralement tubulaire, l'ensemble de branche latérale du stent étant mis en prise avec la bague de montage (47) étant positionné à l'intérieur de la circonférence de la bague de montage (47), et dans lequel la bague de montage (47) comprend également une surface intérieure faisant face à la lumière intérieure, et une surface extérieure dirigée dans le sens opposé dans laquelle l'attache (43) s'étend à travers au moins une portion de la surface extérieure de la bague de montage (47).

10. Stent selon la revendication 1, dans lequel l'attache (43) s'étend au moins partiellement dans une direction circonférentielle autour du stent.

11. Stent selon la revendication 10, dans lequel l'attache (43) forme une boucle complète autour du stent.

12. Stent selon la revendication 1, dans lequel l'attache (43) ne s'étend pas complètement à travers l'ensemble de branche latérale (30) du stent.

13. Stent selon la revendication 1, dans lequel au moins une portion de la paroi généralement tubulaire comprend des éléments d'entretoises de stent interconnectés ayant une surface de stent intérieure qui fait face à la première lumière, et une surface de stent extérieure dirigée dans le sens opposé dans laquelle des cellules de stent définissent des espaces ouverts entre les éléments d'entretoises de stent interconnectés, et l'attache (43) s'étend à travers au moins une cellule de stent.

14. Stent selon la revendication 1, dans lequel l'attache (43) s'étend à partir d'une position plus distale sur l'ensemble de branche latérale (30) du stent vers une position plus distale sur l'ensemble de branche latérale (30) du stent.

15. Stent selon la revendication 1, dans lequel une portion de l'attache (43) est une portion plus faible, étant moins résistante à une force de tension que les autres portions de l'attache (43).
